# EUROPÄISCHE PATENTSCHRIFT

(11) **EP 1 413 570 B1**
(45) Veröffentlichungstag und Bekanntmachung des Hinweises auf die Patenterteilung: **10.05.2006**
(21) Anmeldenummer: 03022338.2
(22) Anmeldetag: 04.10.2003
(51) Int. Cl.: C07C 255/03, A61Q 13/00, C11B 9/00

(54) **5,7,7-Trimethyloctannitril**
5,7,7-Trimethyloctanenitrile
5,7,7-Trimethyloctannitrile

(30) Priorität: 15.10.2002 DE 10247966
(43) Veröffentlichungstag der Anmeldung: 28.04.2004
(73) Patentinhaber: Symrise GmbH & Co. KG, 37603 Holzminden (DE)
(72) Erfinder: Panten, Johannes, 37671 Lüchtringen (DE); Fahlbusch, Karl-Georg, 37671 Höxter (DE); Werner, Matthias, 22587 Hamburg (DE); Sillon, Pascal, 20251 Hamburg (DE)
(74) Vertreter: Eisenführ, Speiser & Partner

(56) Entgegenhaltungen:
- EP-A- 0 017 396
- EP-A- 0 347 596
- EP-B- 0 074 253
- GB-A- 1 523 028

## Beschreibung

Die vorliegende Erfindung betrifft 5,7,7-Trimethyloctannitril, bestimmte Erzeugnisse mit einem Anteil an dieser Substanz (insbesondere Riechstoffkompositionen, parfümierte Artikel und Bleichmittelzusammensetzungen) sowie bestimmte Verwendungen der Substanz.

Trotz einer Vielzahl bereits vorhandener Riechstoffe besteht in der Parfümindustrie auch weiterhin ein genereller Bedarf an neuen Riechstoffen, die über Ihre primären, nämlich geruchlichen Eigenschaften hinaus zusätzliche positive Sekundäreigenschaften besitzen, wie z. B. eine höhere Stabilität unter bestimmten Anwendungsbedingungen, eine höhere Ausgiebigkeit, ein besseres Haftungsvermögen oder dergleichen.

Es ist bereits seit längerem bekannt, dass die chemische Stoffgruppe der Nitrile eine Reihe interessanter Riechstoffe umfasst. Bekannte, kommerzielle Produkte sind Citronellylnitril, Geranylnitril und Cinnamylnitril. Eine Übersicht über Nitrile als Riechstoffe wird gegeben von Vasanti G. Yadav in Perfumery Nitriles and Acetals: Part II, Synthesis and Characteristics of Nitriles, PAFAI J. (1994), 16 (2), 29 - 42. Neuere Veröffentlichungen zu Nitrilen als Inhaltstoffen von Parfümen sind die Patentdokumente US 5,179,222, US 6,180,814 und US 5,521,151. Die letztgenannten Dokumente betreffen Nitrile, die neben ihrer primären geruchlichen Eigenschaft durch eine besonders hohe Stabilität in aggressiven Medien (z. B. wässrigen Lösungen mit besonders hohem oder besonders niedrigem pH-Wert) gekennzeichnet sind.

Es war die primäre Aufgabe der vorliegenden Erfindung, eine chemische Verbindung anzugeben, welche als Riechstoff mit ausgeprägter Iris-Note eingesetzt werden kann und auch in aggressiven Medien eine hohe Stabilität besitzt.

Erfindungsgemäß wird diese Aufgabe gelöst durch Angabe der Verbindung 5,7,7-Trimethyloctannitril. Der Begriff 5,7,7-Trimethyloctannitril umfasst dabei im Rahmen des vorliegenden Textes das R-konfigurierte Enantiomere, das S-konfigurierte Enantiomere sowie (insbesondere racemische) Gemische der Enantiomeren.

Die Strukturformel des 5,7,7-Trimethyloctannitril ist nachfolgend wiedergegeben:

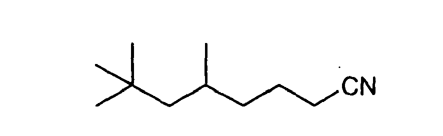

Die geruchlichen Eigenschaften des 5,7,7-Trimethyloctannitril werden beschrieben als: Iris, Vetiver, Irisbutter, holzig, wurzelig.

Die Substanz besitzt neben ihren hervorragenden geruchlichen Eigenschaften auch eine hervorragende Stabilität in aggressiven Medien, wobei hier insbesondere bleach-Systeme (Bleichmittel) zu nennen sind.

Überraschenderweise hat sich gezeigt, dass die erfindungsgemäße Erfindung 5,7,7-Trimethyloctannitril in ihren Eigenschaften sogar der auch unter der Bezeichnung "Irisnitril" bekannten Verbindung 2-Nonennitril CH₃(CH₂)₅CH=CH-CN überlegen ist. 2-Nonennitril besitzt nämlich eine in der Parfümerie unerwünschte geruchliche Stärke, die es zu einem derart aufdringlichen Riechstoff macht, dass in Arctander S., "Perfume and Flavour Chemicals", Vol. I, 1969, Nr. 2362, zu recht ausgeführt wird, dass die Hauptschwierigkeit bei der Promotion dieser Substanz als Duftstoff in ihrer enormen (geruchlichen) Stärke besteht. Das erfindungsgemäße 5,7,7-Trimethyloctannitril ist hingegen weniger aufdringlich und unterscheidet sich von 2-Nonennitril auch in den geruchlichen Nebenaspekten, welche von Parfümeuren besonders geschätzt werden.

Es sei ergänzend darauf hingewiesen, dass aus der von Yadav aaO gegebenen tabellarischen Übersicht über die geruchlichen Charakteristika synthetischer Nitrile, die 48 Substanzen umfasst, überhaupt nur eines, nämlich die Verbindung 3-Methyl-2-Nonennitril (Verbindung 13) als Duftstoff mit einer Irisnote bezeichnet wird.

Während Nitrile häufig einen Geruch besitzen, der dem der korrespondierenden Aldehyde entspricht (vergleiche wiederum Yadav aaO) besteht eine derartige geruchliche Verwandtschaft zwischen dem erfindungsgemäßen 5,7,7-Trimethyloctannitril und seinem korrespondierenden Aldehyd nicht. Es besteht also eine geruchliche Abweichung zwischen Nitril und Aldehyd, die Yadav als "anomalous odour effect" bezeichnet hätte.

Die vorliegende Erfindung betrifft auch Riechstoffkompositionen sowie parfümierte Artikel, die eine sensorisch wirksame Menge an 5,7,7-Trimethyloctannitril umfassen. Parfumeure, die sich mit dem Einsatz der erfindungsgemäßen Substanz befasst haben, stellten fest, dass dieses als Riechstoff eine (noch) bessere Performance als Irisnitril hat, da es im Vergleich mit Irisnitril mit weniger Einsatz mehr Leistung bringt.

Die Menge an 5,7,7-Trimethyloctannitril in einer erfindungsgemäßen Riechstoffkomposition reicht vorzugsweise aus, um die Riechstoffkompositionen in Richtung Iris zu modifizieren und/oder zu verstärken.

Eine Riechstoffkomposition mit einer Irisnote wird erfindungsgemäß hergestellt, indem 5,7,7-Trimethyloctannitril mit üblichen weiteren Bestandteile einer Riechstoffkomposition vermischt wird, wobei das 5,7,7-Trimethyloctannitril in einer Menge eingesetzt wird, die ausreicht, um den Geruch der Riechstoffkomposition in Richtung Iris zu modifizieren und/oder zu verstärken.

Besonders bevorzugt ist der Einsatz der erfindungsgemäßen Substanz in einer Bleichmittelzusammensetzung. Eine erfindungsgemäße Bleichmittelzusammensetzung umfasst dementsprechend
- ein Bleichmittel,
- 5,7,7-Trimethyloctannitril sowie gegebenenfalls
- übliche Zusatzstoffe
wobei das 5,7,7-Trimethyloctannitril in einer Menge vorliegt, die ausreicht, um den Geruch der Bleichmittelzusammensetzung in Richtung Iris zu modifizieren und/oder zu verstärken. Bevorzugt ist dabei, dass das Bleichmittel Chlor und/oder Hypochlorit umfasst.

Weitere bevorzugte Aspekte und Unteraspekte der Erfindung ergeben sich aus den nachfolgenden Beispielen sowie den beigefügten Patentansprüchen:

### Beispiele:

### Parfümkompositionen:

Anwendungsbeispiel für 5,7,7-Trimethyloctannitril:

| | |
|---|---|
| Aldehyde C-10 1%DPG | 8.00 |
| Aldehyde C-11 Lenic 1%DPG | 6.00 |
| Aldehyde C-9 10%DPG | 4.00 |
| Benzylacetate | 65.00 |
| Brahmanol ¹⁾ | 10.00 |
| Cinnamic alcohol | 20.00 |
| Citronellol inaktiv | 90.00 |
| Citronellylacetate | 40.00 |
| Clary sage oil Russia 10%DPG | 6.00 |
| DPG (Dipropylenglycol) | 132.00 |
| Dupical ²⁾ 10%DPG | 3.00 |
| Exaltolide Total ³⁾ | 2.00 |
| Hedione ⁴⁾ | 8.00 |
| Hexenyl Acetate cis-310%DPG | 8.00 |
| Hexyl cinnamic aldehyde | 160.00 |
| Indole 10%DPG | 15.00 |
| Indolene 50⁵⁾ | 10.00 |
| Lemon Oil Italian SFUmatrice | 2.00 |
| Lilial⁶⁾ | 20.00 |
| Linalool Synth. | 90.00 |
| Lyral⁷⁾ | 50.00 |
| Phenylethyl Acetate | 25.00 |
| Phenylethyl Alcohol | 140.00 |
| Phenylethyllsobutyrate | 5.00 |
| Rose oxide I 1%DPG | 6.00 |
| Sandalwood oil East India | 5.00 |
| Terpineol Pure | 60.00 |
| 5,7,7-Trimethyloctannitril | 10.00 |
| | 1000.00 |

| | |
|---|---|
| 1) Handelsname der Fa. Dragoco, Holzminden, D | |
| 2) Handelsname der Fa. Quest, Ashford, GB | |
| 3),4) Handelsname der Fa. Firmenich, Genf, CH | |
| 5),6) Handelsname der Fa. Givaudan, Zürich, CH | |

7) Handelsname der Fa. IFF, New Jersey, US

Geruchsbeschreibung: blumig, Maiglöckchen, sehr natürlich, sehr weich, Iris

### Bleichmittelzusammensetzung:

| | |
|---|---|
| Laurinsäure Natriumsalz (als 30%-Lsg.) | 1,5 |
| Cocosstearyldimethylaminoxid | 3,5 |
| Hypochlorit (als 13%-Lsg.) | 40,0 |
| Natriumhydroxid (als 30%-Lsg.) | 2,5 |
| Wasser | 52,4 |
| 5,7,7-Trimethyloctannitril | 0.1 |
| | 100,0 |

### Herstellung:

Die Herstellung von 5,7,7-Trimethyloctannitril erfolgt in an sich bekannter Weise. So kann vorteilhaft mittels einer Knoevenagel-Reaktion zunächst aus Isononylaldehyd und Cyanessigsäure Z/E-5,7,7-Trimethyl-2(3)-octennitril hergestellt werden, das geruchlich überraschenderweise völlig anders zu charakterisieren ist als die erfindungsgemäße Verbindung, vgl. Yadov aaO, Verbindung 8.

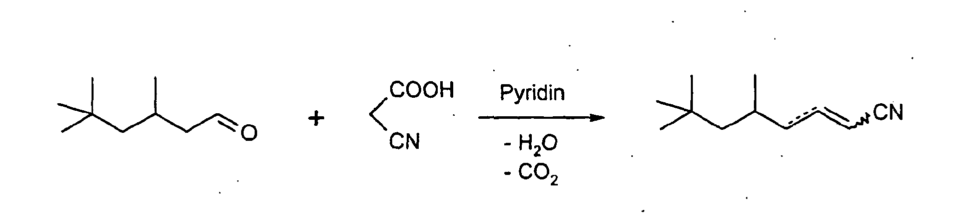

Die anschließende Hydrierung zum gesättigten Nitril erfolgt dann vorteilhaferweise mit Pd/C:

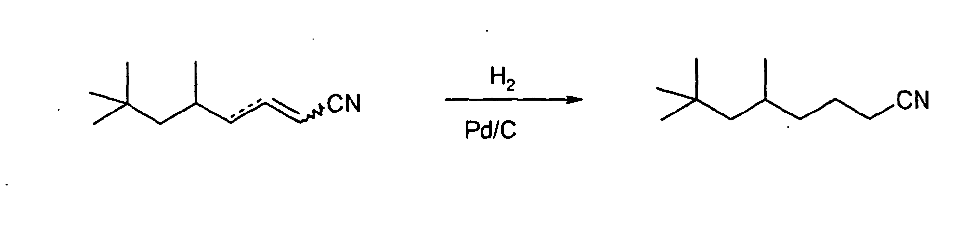

### Herstellvorschrift:

### 1. Stufe: Darstellung von E/Z-5,7,7-Trimethyl-2(3)-octennitril

300g Toluol, 255 g (3 mol) Cyanessigsäure und 426 g (3 mol) Isononylaldehyd werden in der angegebenen Reihenfolge vorgelegt und unter Rühren auf 55-60°C erhitzt. Dann werden ohne weiteres Erhitzen innerhalb von 15 min. 64 g Pyridin zugetropft. Die Temperatur steigt auf 65°C. Anschließend wird innerhalb von 45 min. zum Sieden erhitzt und 4 h am Wasserabscheider das Reaktionswasser abgetrennt. Es wird auf 70-75°C abgekühlt und bei 70°C mit 2x 150 g Wasser, 2x 150 g 10% H₂SO₄ und 1 x 150 g NaCl-Lsg. gewaschen. Anschließend wird destilliert.

Ausbeute an E/Z-5,7,7-Trimethyl-2(3)-octennitril (4 Isomere): 270,7 g (55%)

### 2. Stufe: Hydrierung zu 5,7,7-Trimethyloctannitril

270 g (1,6 mol) E/Z-5,7,7-Trimethyl-2(3)-octennitril, 1,8 I Ethanol und 3,24 g Pd/C werden 10 h bei 30°C und 40 bar unter Wasserstoff gerührt. Die Vollständigkeit der Hydrierung wird mittels GC überprüft.

Anschließend wird das Lösungsmittel abgezogen und an einer 20 cm GFKK fraktioniert.

Ausbeute: 210,1 g (77 %).

### Spektroskopische Daten:

### 5,7,7-Trimethyloctannitril:

^{**1**}**H-NMR (CDCl**_{**3**}**, 300 MHz, TMS= 0 ppm):** δ = 0,90 (s, 9 H); 0,93 (d, 3 H, *J*= 6,5 Hz); 1,08 (dd, 1 H, *J*= 14,0 und 6,2 Hz); 1,21 (dd, *J*= 14,0 und 3,5 Hz, 1 H); 1,25 - 1,55 (m, 3 H); 1,55 - 1,72 (m, 2 H); 2,32 (t, 2 H, *J*= 7,14 Hz).
^{**13**}**C-NMR (CDCl**_{**3**}**, 75 MHz)**: δ = 17,4 (t); 22,4 (q); 23,2 (t); 28,7 (d); 30,0 (3 q); 31,0 (s); 38,4 (t); 51,0 (t); 119,8 (s).

**MS (m/e, %):** 166 (M⁺,0); 152 (58); 110 (13); 96 (23); 69 (13); 57 (100); 56 (11); 55 (13); 41 (26).

### Vorstufe E/Z-5,7,7-Trimethyl-2(3)-octennitril:

### MS (m/e,%):

**Isomer 1:** 165 (M,0); 150 (60); 108 (20); 94 (23); 67 (40); 57 (100); 41 (38).

**Isomer 2:** 165 (M,1); 150 (20); 109 (25); 94 (50); 71 (40); 57 (100); 41 (30).

**Isomer 3:** 165 (M,0); 150 (20); 123 (20); 108 (20); 99 (25); 67 (30); 57 (100); 41 (30).

**Isomer 4:** 165 (M,15); 150 (20); 109 (40); 94 (15); 71 (20); 57 (100); 41 (40).

### IR:

**Isomer 1**: ν (cm⁻¹) = 2963,21; 2915,05; 1472,96; 1371,26; 1241,54.

**Isomer 2:** ν (cm⁻¹) = 2964,67; 2914,47; 1471,77; 1371,36.

**Isomer 3:** ν (cm⁻¹) = 2963,98; 2918,76; 2228,71; 1634,35; 1473,54; 1371,04; 966,55.

**Isomer 4**: ν (cm⁻¹) = 2964,57; 2916,06; 1472,04; 1370,03; 971,37.

## Patentansprüche

1. 5,7,7-Trimethyloctannitril

2. Riechstoffkomposition oder parfümierter Artikel, umfassend eine sensorisch wirksame Menge an 5,7,7-Trimethyloctannitril.

3. Riechstoffkomposition nach Anspruch 2, umfassend eine Menge an 5,7,7-Trimethyloctannitril, die ausreicht, um die Riechstoffkomposition in Richtung Iris zu modifizieren und/oder zu verstärken.

4. Verwendung von 5,7,7-Trimethyloctannitril als Riechstoff.

5. Verfahren zur Herstellung einer Riechstoffkomposition mit einer Irisnote, mit folgendem Schritt:
- Vermischen von 5,7,7-Trimethyloctannitril mit üblichen Bestandteilen einer Riechstoffkomposition, wobei das 5,7,7-Trimethyloctannitril in einer Menge eingesetzt wird, die ausreicht, um den Geruch der Riechstoffkomposition in Richtung Iris zu modifizieren und/oder zu verstärken.

6. Bleichmittelzusammensetzung, umfassend
- ein Bleichmittel,
- 5,7,7-Trimethyloctannitril sowie gegebenenfalls
- übliche Zusatzstoffe
wobei das 5,7,7-Trimethyloctannitril in einer Menge vorliegt, die ausreicht, um den Geruch der Bleichmittelzusammensetzung in Richtung Iris zu modifizieren und/oder zu verstärken.

7. Bleichmittelzusammensetzung nach Anspruch 6, wobei das Bleichmittel Chlor und/oder Hypochlorit umfasst.

## Claims

1. 5,7,7-Trimethyloctanenitrile.

2. Fragrance composition or perfumed article containing a sensorially effective amount of 5,7,7-trimethyloctanenitrile.

3. Fragrance composition according to claim 2, containing an amount of 5,7,7-trimethyloctanenitrile which is sufficient to modify and/or to intensify the fragrance composition in the iris direction.

4. Use of 5,7,7-trimethyloctanenitrile as a fragrance substance.

5. Process for the preparation of a fragrance composition having an iris note, with the following step:
- Mixing 5,7,7-trimethyloctanenitrile with conventional constituents of a fragrance composition, the 5,7,7-trimethyloctanenitrile being used in an amount which is sufficient to modify and/or intensify the odour of the fragrance composition in the iris direction.

6. Bleaching agent composition, comprising
- a bleaching agent,
- 5,7,7-trimethyloctanenitrile and optionally
- conventional additives,
the 5,7,7-trimethyloctanenitrile being present in an amount which is sufficient to modify and/or to intensify the odour of the bleaching agent composition in the iris direction.

7. Bleaching agent composition according to claim 6, wherein the bleaching agent comprises chlorine and/or hypochlorite.

## Revendications

1. 5,7,7-triméthyloctanenitrile.

2. Composition de substances odorantes ou article parfumé comprenant une quantité efficace du point de vue sensoriel de 5,7,7-triméthyloctanenitrile.

3. Composition de substances odorantes selon la revendication 2 comprenant une quantité de 5,7,7-triméthyloctanenitrile qui est suffisante pour modifier et/ou renforcer la composition de substances odorantes dans la direction iris.

4. Utilisation du 5,7,7-triméthyloctanenitrile comme substance odorante.

5. Procédé de production d'une composition de substances odorantes à note d'iris avec l'étape suivante :
- mélange du 5,7,7-triméthyloctanenitrile avec des constituants courants d'une composition de substances odorantes, où le 5,7,7-triméthyloctanenitrile est utilisé en une quantité qui est suffisante pour modifier et/ou renforcer l'odeur de la composition de substances odorantes dans la direction iris.

6. Composition d'agent de blanchiment comprenant
- un agent de blanchiment,
- du 5,7,7-triméthyloctanenitrile et éventuellement
- des additifs courants
où le 5,7,7-triméthyloctanenitrile est présent en une quantité qui est suffisante pour modifier et/ou renforcer l'odeur de la composition d'agent de blanchiment dans la direction iris.

7. Composition d'agent de blanchiment selon la revendication 6 où l'agent de blanchiment comprend du chlore et/ou de l'hypochlorite.
